# EUROPEAN PATENT SPECIFICATION

(11) **EP 3 294 815 B1**
(45) Date of publication and mention of the grant of the patent: **02.09.2020**
(21) Application number: 16796921.1
(22) Date of filing: 04.05.2016
(51) Int. Cl.: C09D 4/02, C09D 11/10, C09D 11/101, C09D 11/102, C09D 11/107, C09D 11/30, C07C 229/30

(54) **ENERGY CURABLE INKJET INKS AND COATING COMPOSITIONS**
ENERGIEHÄRTBARE TINTENSTRAHLTINTEN UND BESCHICHTUNGSZUSAMMENSETZUNGEN
ENCRES POUR JET D'ENCRE DURCISSABLES PAR RAYONS D'ÉNERGIE ET COMPOSITIONS DE REVÊTEMENT

(30) Priority: 15.05.2015 US 201562161933 P
(43) Date of publication of application: 21.03.2018
(73) Proprietor: Sun Chemical Corporation, Parsippany, NJ 07054 (US)
(72) Inventor: ILLSLEY, Derek, Frome Bath BA11 4JB (GB)
(74) Representative: Carpmaels & Ransford LLP
(86) International application number: PCT/US2016/030659
(87) International publication number: WO 2016/186838

(56) References cited:
- EP-A1- 1 876 166
- WO-A2-03/011990
- WO-A2-2008/077045
- US-A1- 2007 004 815
- US-A1- 2009 098 304
- US-A1- 2009 099 278
- US-A1- 2009 099 279
- US-A1- 2009 284 698
- US-A1- 2009 318 611
- US-A1- 2012 308 734

## Description

### CROSS REFERENCE TO RELATED APPLICATIONS

This application claims priority to U.S. Provisional Patent Application Serial No. 62/161,933 filed May 15th, 2015.

### FIELD OF THE INVENTION

The present invention is directed to energy curable inkjet ink or coating compositions comprising at least one aminoacrylate that when cured exhibit low migration and improved adhesion to plastic surfaces and thus are useful for sensitive applications such as food packaging, pharmaceutical packaging and/or cosmetics and personal care packaging.

### BACKGROUND OF THE INVENTION

The present invention relates to energy curable inkjet ink or coating compositions comprising an aminoacrylate that is prepared by the Michael addition reaction of a polyacrylate with an alkanolamine. Furthermore the ink and coating compositions limit the amount of total acyl phosphine oxide photoinitator and monofunctional monomer content therein.

EP 2 671 722 is directed to industrial inkjet printing methods on packaging materials for foodstuffs and pharmaceutical compounds and liquids and describes the use of an amine modified polyetheracrylate adhesion promoter in the preparation of ultra-violet (UV) curable inkjet fluids. The fluids also contain high concentrations of silicone acrylate to increase adhesion.

WO2014/126720 is directed to pigmented free radical UV curable inkjet liquid compositions for the printing of food packaging materials and describes that cure response and a further lowering of migratable monomer can be achieved by the inclusion of suitable co-initiators such as acrylated amines and aminobenzoates.

US 3,844,916, US 3,914,165 and US 3,925,349 are all directed to radiation curable non-gelled Michael addition reaction products that are obtained by reacting amines comprising at least one amino hydrogen atom with a stoichiometric excess of ethylenic material comprising a polyacrylate.

US20110159251 describes radiation curable ink compositions that contain a phenoxyethyl acrylate, a multifunctional acrylate and a pigment. Furthermore the composition may contain an amino acrylate which can be an adduct of hexandiol diacrylate (HDDA) and ethanolamine. However, the compositions use significant quantities of a monofunctional monomer, such as greater than 20% of either phenoxyethyl acrylate (PHEA) or a blend of PHEA with N-vinyl caprolactam (NVC) and high amounts of acyl phosphine oxide photoinitator.

WO2015/036615 describes radiation curable inkjet compositions for food packaging which can comprise polymeric or polymerizable aminobenzoate type co-initiators and may also include thioxanthone type photoinitiators.

Finally JP2013159707, JP2012188613, JP2011213931, JP2011213934, and JP2011213933 are all directed to energy curable inkjet compositions which contain a large proportion of monofunctional monomer.

EP1876166 relates to amino(meth)acrylates obtained from the reaction of amines with a mixture of epoxy(meth)acrylates and (meth)acrylated diluents. WO03011990 relates to the field of liquid pigment inks, in particular those intended for ink jet printing. US2009/0284698 relates to an ink composition that improves a contrast ratio according to dispersion of fine pigment. US2009/0099278 and US2009/0099279 relate to special acrylated polyaminoamides and to their use for radiation curable coatings. WO2008/077045 relates to coating composition adhesion promotion additives.

### SUMMARY OF THE INVENTION

The present invention provides an energy curable inkjet ink or coating composition comprising
a) at least one aminoacrylate and
b) and at least one photoinitator; wherein the photoinitiator comprises a difunctional, multifunctional, oligomeric and/or polymeric photoinitiator;
wherein the total photoinitiator content is less than 12.0% by weight and the total acyl phosphine oxide photoinitator content is less than 4.0% by weight;
wherein the ink or coating composition comprises less than 10.0% by weight of monofuntional monomer and
wherein the aminoacrylate is prepared by the Michael addition reaction of a polyacrylate with an alkanolamine having the formula:

HN(R¹)(CₙH₂ₙOH)

wherein R¹ is H or (CₘH₂ₘX);
X is H or OH;
m is 1 to 6 and n is 1 to 6.

Furthermore the present invention also provides a coated article comprising a cured ink or coating composition according to anyone of the preceding claims on the surface of the article.

Finally the present invention also provides a process for preparing a coated article comprising
a) applying the ink or coating composition to the surface of the article and
b) curing the ink or coating composition.

These and other objects, advantages, and features of the invention will become apparent to those persons skilled in the art upon reading the details of the methods and formulations as more fully described below.

### BRIEF DESCRIPTION OF THE DRAWING

Figure 1 shows contaminants from ink which have diffused into the contact surface of the package which can then leach into the food causing a potential contamination issue.
Figure 2 is a Cross Hatch Test according to ASTM D3359-09 showing a score of 0 (total adhesion loss) to 5 (no adhesion loss).
Figure 3 shows a second adhesion test involves folding the print.

### DETAILED DESCRIPTION OF THE INVENTION

The present invention is directed to inks and compositions that use Michael reaction products based on the reaction of polyacrylates with alkanolamines that when cured exhibit low migration, improve the adhesion to plastic substrates, exhibit faster cure and lower odor.

Accordingly the present invention provides an energy curable inkjet ink or coating composition which contains at least one aminoacrylate prepared by the Michael addition reaction of a polyacrylate with an alkanolamine and contains less that 12.0% by weight of total photoinitiator.

Furthermore the ink or coating compositions contain less than 4.0% by weight of acyl phosphine oxide photoinitator and contain less than 10.0% by weight of monofuntional monomer.

It has been found that the inks or coating compositions of the present invention when cured exhibit lower levels of odor than from comparable inks comprising aminoacrylates based on aliphatic amines, such as diethylamine and dipropylamine. This is a distinct advantage for inks intended for the printing of sensitive applications such as food packaging, pharmaceutical packaging and/or cosmetics and personal care packaging.

Preferably the aminoacrylate is derived from the Michael addition reaction product of a primary or secondary alkanolamine with a stoichiometric excess of a polyacrylate.

Typically the inks and coating compositions contain between 1.0 to 50.0% by weight, preferably between 2.0 to 40% by weight and advantageously between 5.0 to 20.0% by weight of aminoacrylate.

Furthermore the amount of aminoacrylate in the ink and coating compositions preferably contain sufficient aminoacrylate such that the amine value of the ink or coating composition is greater than 1.0 mgKOH/g, more preferably greater than 3.0 mgKOH/g, and advantageously greater than 5.0 mgKOH/g.

Usually the polyacrylate is selected from dipropyleneglycol diacrylate, tripropyleneglycol diacrylate, hexanediol diacrylate, ethoxylated (2 moles) hexanediol diacrylate, ethoxylated (3 moles) hexanediol diacrylate, 3-methylpentanediol diacrylate, decanediol diacrylate, dodecanediol diacrylate, propoxylated (2 moles) neopentyl glycol diacrylate, trimethylolpropane triacrylate, ethoxylated (3 moles) trimethylolpropane triacrylate, ethoxylated (4 moles) trimethylolpropane triacrylate, ethoxylated (5 moles) trimethylolpropane triacrylate, propoxylated (3 moles) trimethylolpropane triacrylate, propoxylated (4 moles) trimethylolpropane triacrylate, propoxylated (5 moles) trimethylolpropane triacrylate propoxylated (3 moles) glyceryl triacrylate, propoxylated (4 moles) glyceryl triacrylate, propoxylated (5 moles) glyceryl triacrylate, pentaerythritol triacrylate, pentaerythritol tetraacrylate, ethoxylated (4 moles) pentaerythritol tetracrylate, ethoxylated (5 moles) pentaerythritol tetracrylate, ethoxylated (6 moles) pentaerythritol tetracrylate, ethoxylated (7 moles) pentaerythritol tetracrylate, dipentaerythritol pentaacrylate, ethoxylated (5 moles) dipentaeryhtritol pentaacrylate, ethoxylated (6 moles) dipentaeryhtritol pentaacrylate, ethoxylated (7 moles) dipentaeryhtritol pentaacrylate, ethoxylated (8 moles) dipentaeryhtritol pentaacrylate, ethoxylated (9 moles) dipentaeryhtritol pentaacrylate, ditrimethylolpropane tetraacrylate, ethoxylated (4 moles) ditrimethylolpropane tetraacrylate, ethoxylated (5 moles) ditrimethylolpropane tetraacrylate, ethoxylated (6 moles) ditrimethylolpropane tetraacrylate, ethoxylated (7 moles) ditrimethylolpropane tetraacrylate, diethyleneglycol diacrylate, triethyleneglycol diacrylate, tricyclodecanedimethanol diacrylate (TCDDMDA), ethoxylated 3 and/or bisphenol A diacrylate.

Advantageously the polyacrylate is selected from hexanediol diacrylate, 3-methylpentanediol diacrylate, dipropylene glycol diacrylate, tripropyleneglycol diacrylate, ethoxylated (3 moles) trimethylolpropane triacrylate, propoxylated (3 moles) glyceryl triacrylate, neopentyl glycol diacrylate, decanediol diacrylate, ethoxylated (2 moles) hexanediol diacrylate, propoxylated (2 moles) neopentyl glycol diacrylate and/or propoxylated (3 moles) trimethylolpropane triacrylate.

Although polyacrylate monomers are preferred, the inks and coating compositions may include those Michael addition reaction products formed by the reaction of alkanolamines with ethylenically unsaturated oligomers and polymers such as; polyester acrylates, epoxy acrylates, polyurethane acrylates and/or acrylic acrylates.

Typically the alkanolamines may be selected from methanolamine, ethanolamine, diethanolamine, propanolamine, dipropanolamine, isopropanolamine, butanolamine, isobutanolamine, N-methylethanolamine, N-ethylethanolamine, N-methylpropanolamine and/or N-phenylethanolamine.

Preferably alkanolamine is selected from ethanolamine, diethanolamine, propanolamine and/or N -methylethanolamine.

Usually the aminoacrylates may be selected from any commercially available aminoacrylates such as Ebecryl 81, LEO10551, LEO10553 and P116 (ex. Allnex) and CN3715, CN3705, CN3735 and CN381 (ex. Sartomer), Ebecryl 7100, Ebecryl 80, Ebecryl 83, LEO10552, Ebecryl LEO10552 (ex. Allnex), CN550, CN3755, CN554 (ex. Sartomer) and Photomer 4711 and Photomer 4755 (ex. IGM).

In addition it has been found that the use of aminoacrylates as opposed to aminobenzoates as co-initiators is advantageous given that aminobenzoate co-initiators absorb strongly between 260 and 340nm in the UV spectrum and as such they can attenuate the intensity of UV light in this region. Therefore the amount of UV light available for photoinitiators absorbing in this region to generate free radicals is restricted.

Consequently that amount of photoinitiators in the inks and coating compositions which absorb in the 260-340nm range of the electromagnetic spectrum which include benzophenone types, hydroxyalkyl phenone ('hydroxy ketone') types, aminoalkylphenone types and acylphosphine oxide types can be reduced.

The use of aminoacrylates in the inks and compositions is also advantageous in that they can act as a proton donor, which facilitates the effective action of type II H-abstracting photoinitiators such as the benzophenone and thioxanthone types. Furthermore the aminoacrylates also reduce some of the deleterious effects of oxygen inhibition of the free radical curing, in air, of ethylenically unsaturated monomers and oligomers.

This is particularly advantageous given that typically high amounts of free radical photoinitators, up to and exceeding 10.0% by weight of the total ink or coating composition are employed in injet fluids due to the low viscosity of the fluids that allows oxygen from the atmosphere to readily diffuse into the ink film before and during the energy-curing process.

Thus the inks and coating compositions contain less than 12.0% by weight of total photoinitator, typically less than 8.0% by weight, preferably less than 6.0% by weight and advantageously less than 5.0% by weight of total photoinitiator.

Preferably the inks and coating compositions contain low migration photoinitiators and in particular photoinitiators having recognized low migration potential which are listed Group 1A and Group 1B of the current and future editions of EuPIA's (European Printing Ink Association) Suitability List of Photo-initiators for Low Migration UV Printing Inks and Varnishes.

The photoinitiators are the difunctional, multifunctional, oligomeric, polymeric and/or polymerizable types.

Particularly favored photoinitiators include phenyl bis(2,4,6-trimethylbenzoyl) phosphine oxide ('Irgacure 819', Cas.No.162881-26-7), difunctional and oligomeric hydroxyl ketone types such as Irgacure 127 (Cas.No.474510-57-1) and Esacure KIP160 (Cas.No.71868-15-0). 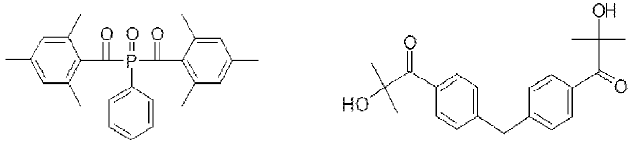 Irgacure 819 (ex. BASF) Irgacure 127 (ex. BASF) 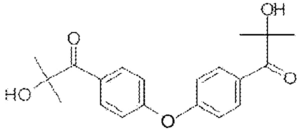 Esacure KIP160 (ex. Lamberti)

Advantageously polymeric photoinitiators, wherein the photoinitiator moieties are chemically attached to a polymeric core are used.

An example of a polymeric photoinitiator is Omnipol TX (ex. IGM Resins, CAS No.: 813452-37-8), which has the following structure;

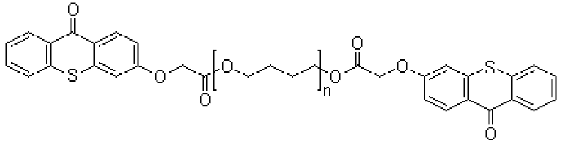

Other commercially available polymeric thioxanthones include Genopol TX-1 (ex.Rahn) and Speedcure 7010 (ex. Lambson).

Where polymeric photoinitiators are used in the ink and coating compositions it is preferred that their total concentration is less than 4.0% by weight, preferably less than 3.0% by weight (w/w) and advantageously less than 2.0% by weight.

Finally other similar polymeric photoinitiators such as those comprising benzophenone, aminoketone, or other photoinitiator groups may also be used.

It has also been found that limiting the concentration of the acyl phosphine oxide photoinitiator also limits the amount of photodecomposition by-products that could cause contamination of the environment.

Where phosphine oxide photoinitiators, such as phenyl bis(2,4,6-trimethylbenzoyl) phosphine oxide (Cas.No. 162881-26-7), are used in low migration UV curable inkjet compositions, concentrations greater than 2.5% (w/w) of the total ink composition are typically employed.

In particular, one photoinitiator class where it is useful to restrict the concentration in the ink composition is the acylphosphine oxide types. A particular acylphosphine oxide photoinitiator that is useful in the preparation of UV curable inkjet fluids, and especially those for the printing of food packaging substrates and the like is phenyl bis(2,4,6-trimethylbenzoyl) phosphine oxide (Cas.No. 162881-26-7) which is available commercially as Irgacure 819 (ex. BASF).

This photoinitiator has a specific migration limit of 3.3mg/Kg(food). However, two principal photodecomposition by-products from this photoinitiator are mesitaldehyde (2,4,6-trimethylbenzaldehyde) and 2,4,6-trimethylbenzoic acid.

Mesitaldehyde, especially, is highly prone to migrating from cured ink films and causing contamination issues. Therefore, the compositions of the present invention achieve acceptable cure with concentrations of bis(2,4,6-trimethylbenzoyl) phosphine oxide of less than 2.5% by weight of the total ink composition, more preferably less than 2.0% by weight, and advantageously less than 1.0% by weight.

When radiation curable inks and varnishes are applied to the (non-contact) surface of primary or secondary packaging intended for foodstuffs, then any contamination from the package impacting the foodstuff should fall within the guidelines set out by Article 3 of Regulation (EC) No 1935/2004, as recommended by EUPIA, requiring that materials and articles in contact with food.

In particular, EUPIA has recommended that Article 3 of this provision be followed when producing printed matter for food packaging and has produced a detailed guideline for the selection of raw materials intended for printing inks for food packaging, along with guidelines on the testing of printed matter to ensure that regulatory requirements are achieved. Furthermore, it is a requirement of the inks of this invention, that when they are cured under the action of UV light that the level of contaminants arising from the cured ink film and reaching the foodstuff should fall below the specific migration limit for any material, in the instances where specific migration limit (SML) exist for those compounds. Wherein no specific SML exists for a compound then general limits apply.

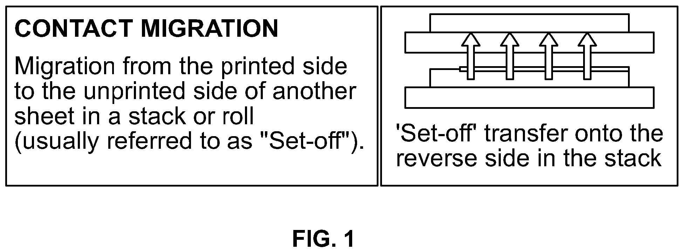

Additionally EUPIA also provides guidelines on how to measure the potential level of migratables arising from printed matter. For inks applied to the non-food contact surface of packaging (i.e. the outer surface), whether that be to the primary packaging or secondary packaging (labels and sleeves) then the most likely route for migratable species from the ink contaminating the foodstuff is by what is known as set-off migration (see figure 1). This is where printed matter is stacked or reeled prior to it being filled with food. Thus, the ink comes into contact with what will be the food-contact surface of the package and migratable components of the ink can diffuse into this surface. When the package is then filled with foodstuff, the contaminants from the ink which have diffused into the contact-surface of the package can then leach into the food causing a potential contamination issue.

Thus, any UV-curable inkjet fluid which is applied to either the primary or secondary packaging of foodstuff should not result in contamination of that foodstuff at levels exceeding the limits detailed above.

In particular bis(2,4,6-trimethylbenzoyl) phosphine oxide and other acylphosphine oxide photoinitiators, although the parent photoinitiator has a relatively high specific migration limit of 3.3mg/Kg (or 3,300ppb in any foodstuff), its photodecomposition by-products are currently bounded by the 10ppb limit.

It has been found that to guarantee that this limit, especially for mesitaldehyde, is not breached, then the inkjet fluids should contain preferably no more than 1.0% by weight, and certainly no more than 2.0% by weight of acylphosphine oxide photoinitiators.

Thus the inks and coating compositions according to present invention provides excellent UV cure response and low levels of unbound, and hence migratable, monomer from cured ink films with concentrations of phosphine oxide photoinitiator of less than 2.5% by weight of the total composition, preferably less than 2.0% by weight and advantageously less than 1.0% by weight(w/w).

In addition to the above mentioned problems adhesion of UV curable inkjet fluids to plastic surfaces can be problematic, especially when the polymerizable component of the composition consists of significant quantities of multifunctional monomers such as HDDA (hexanediol diacrylate), DPGDA (dipropyleneglycol diacrylate) and/or TMPTA (trimethylolpropane triacrylate).

Conventionally, monofunctional monomers such as NVC (N-vinyl caprolactam) and PHEA (phenoxyethyl acrylate) are used to achieve the required level of adhesion to various plastic surfaces.

However, when energy-curable inkjet fluids for the food packaging market or other sensitive produce are used the amount of monofunctional monomers must be limited, or indeed excluded, so as to minimise the risk of unreacted monomer migrating from the cured ink film and causing contamination of packaged foodstuff.

Other sensitive produce packaging includes that for pharmaceutical packaging, toys, cosmetics and/or personal care packaging. Such packaging may include substrates containing PET, LDPE, HDPE, LLDPE, polyamide, and/or CPP and in particular, substrates that are difficult to adhere to include heavier gauge plastic films and structures used in the manufacture of bags, tubes, pouches and bottles.

Consequently to achieve the required low levels of migratable species that can elute from cured inkjet films it is necessary to use polymerizable compositions based largely on multifunctional monomers and oligomers which reduces the ink or coating compositions capability of adhering to plastic surfaces.

It has surprisingly been found that the use the particular aminoacrylate according to the present invention, in conjunction with the type and amount of photoinitiator can deliver significant improvements in adhesion and migration of multifunctional inkjet fluids with low amounts of multifunctional monomer.

Consequently it is a requirement the inks and coating compositions contain less than 10.0% by weight of any monofunctional monomer, more preferably less than 5.0% by weight of monofunctional monomer, and more preferably are essentially free of any intentionally added monofunctional monomer.

Typically the inks and coating compositions further comprise multifunctional ethylenically unsaturated monomers such as 1,3-butylene glycol diacrylate; 1,4-butanediol diacrylate; neopentyl glycol diacrylate; ethoxylated neopentyl glycol diacrylate; propoxylated neopentyl glycol diacrylate; 2-methyl-1,3-propanediyl ethoxy acrylate; 2-methyl-1,3-propanediol diacrylate; ethoxylated 2-methyl-1,3-propanediol diacrylate; 3 methyl 1,5-pentanediol diacrylate; 2-butyl-2-ethyl-1,3-propanediol diacrylate; 1,6-hexanediol diacrylate; alkoxylated hexanediol diacrylate; ethoxylated hexanediol diacrylate; propoxylated hexanediol diacrylate; 1,9-nonanediol diacrylate; 1,10 decanediol diacrylate; ethoxylated hexanediol diacrylate; alkoxylated hexanediol diacrylate; diethyleneglycol diacrylate; triethylene glycol diacrylate; tetraethylene glycol diacrylate; polyethylene glycol diacrylate; propoxylated ethylene glycol diacrylate; dipropylene glycol diacrylate; tripropyleneglycol diacrylate; polypropylene glycol diacrylate; poly (tetramethylene glycol) diacrylate; cyclohexane dimethanol diacrylate; ethoxylated cyclohexane dimethanol diacrylate; alkoxylated cyclohexane dimethanol diacrylate; polybutadiene diacrylate; hydroxypivalyl hydroxypivalate diacrylate; tricyclodecanedimethanol diacrylate; 1,4-butanediylbis[oxy(2-hydroxy-3,1-propanediyl)]diacrylate; ethoxylated bisphenol A diacrylate; propoxylated bisphenol A diacrylate; propoxylated ethoxylated bisphenol A diacrylate; ethoxylated bisphenol F diacrylate; 2-(2-Vinyloxyethoxy)ethyl acrylate; dioxane glycol diacrylate; ethoxylated glycerol triacrylate; glycerol propoxylate triacrylate; pentaerythritol triacrylate; trimethylolpropane triacrylate; caprolactone modified trimethylol propane triacrylate; ethoxylated trimethylolpropane triacrylate; propoxylated trimethylol propane triacrylate; tris (2-hydroxy ethyl) isocyanurate triacrylate; e-caprolactone modified tris (2-hydroxy ethyl) isocyanurate triacrylate; melamine acrylate oligomer; pentaerythritol tetraacrylate; ethoxylated pentaerythritol tetraacrylate; di-trimethylolpropane tetra acrylate; dipentaerythritol pentaaacrylate; dipentaerythritol hexaaacrylate and/or ethoxylated dipentaerythritol hexaacrylate A, wherein term ethoxylated refers to chain extended compounds through the use of ethyleneoxide, propoxylated refers to chain extended compounds through the use of propylene oxide, and alkoxylated refers to chain extended compounds using either or both ethyleneoxide and propylene oxide.

Preferably the ink and coating compositions include highly alkoxylated monomers such as ethoxylated or propoxylated trimethylolpropane triacrylates, polyethylene glycol diacrylates and polypropylene glycol diacrylates.

Furthermore it is desirable that the average degree of alkoxylation per polymerizable (especially acrylate) moiety in the monomer/oligomer is greater than 2.0 and preferably equal to or greater than 3.0.

In the case of ethoxylated TMPTA this means that a+b+c in the chemical structure below should be greater than 6.0 and preferably equal to or greater than 9.0.

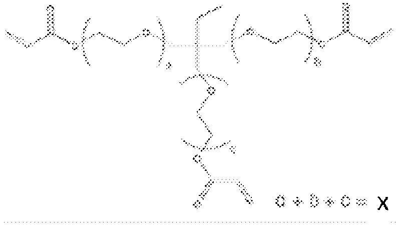

Equivalent methacrylate compounds are also capable of being used.

The inclusion of such monomers has been found to result in achieving substantially reduced levels of unbound, unreacted monomer/oligomer in the cured ink/coating film which might otherwise be free to migrate from the cured ink/coating film and hence cause contamination issues whether that be with food packaging or other sensitive applications.

Advantageously the inks and coating compositions include hybrid monmers containing both (meth)acrylate and vinyl ether polymerizable groups in the monomer molecule. A particularly preferred monomer is 2-(2-vinyloxyethoxy)ethyl acrylate ('VEEA').

The inks and coating compositions may further include other functional monomers which include cyclic lactam such as N-vinyl Caprolactam, N-vinyl oxazolidinone and N-vinyl pyrrolidone, and secondary or tertiary acrylamides such as acryloyl morpholine, diacetone acrylamide, N-methyl acrylamide, N-ethyl acrylamide N-isopropyl acrylamide, N-t.butyl acrylamide, N-hexyl acrylamide, N-cyclohexyl acrylamide, N-octyl acrylamide, N- t.octyl acrylamide N-dodecyl acrylamide, N-benzyl acrylamide, N-(hydroxymethyl)acrylamide, N-isobutoxymethyl acrylamide, N- butoxymethyl acrylamide, N,N-dimethyl acrylamide, N,N-diethyl acrylamide, N,N-propyl acrylamide, N,N-dibutyl acrylamide, N,N-dihexyl acrylamide, N,N-dimethylamino methyl acrylamide, N,N-dimethylamino ethyl acrylamide, N,N-dimethylamino propyl acrylamide, N,N-dimethylamino hexyl acrylamide, N,N-diethylamino methyl acrylamide, N,N-diethylamino ethyl acrylamide, N,N-diethylamino propyl acrylamide, N,N-dimethylamino hexyl acrylamide, and/or N,N'-methylenebisacrylamide.

Additionally the ink and coating compostions of the present invention may also comprise any type or blend of radiation-curable oligomers such as polyurethane acrylates, polyester acrylates, polyether acrylates and/or epoxy acrylates.

The ink and coating compositions usually have a viscosity of less than 15.0mPa.s at 50°C, and advantageously a viscosity of less than 12.0mPa.s at 50°C.

The ink and coating compositions may also include pigments and/or dyes such as azo dyes, anthraquinone dyes, xanthene dyes, azine dyes and combinations thereof.

Typically the organic pigments may be selected from Pigment Yellow Numbers 12, 13, 14, 17, 74, 83, 114, 126, 127, 174, 188; Pigment Red Numbers 2, 22, 23, 48:1, 48:2, 52, 52:1, 53, 57:1, 112, 122, 166, 170, 184, 202, 266, 269; Pigment Orange Numbers 5, 16, 34, 36; Pigment Blue Numbers 15, 15:3, 15:4; Pigment Violet Numbers 3, 23, 27; and/or Pigment Green Number 7. Inorganic pigments may be one of the following non-limiting pigments: iron oxides, titanium dioxides, chromium oxides, ferric ammonium ferrocyanides, ferric oxide blacks, Pigment Black Number 7 and/or Pigment White Numbers 6 and 7.

Additionally the ink and coating compositions may also contain other components which enable them to perform in their intended application. These other components include, stabilizers, wetting aids, slip agents, inert resins, antifoams, fillers, rheological aids and/or amine synergists.

Furthermore the ink and coating compositions may optionally contain an acrylic polymer or copolymer which is dissolved into the ink or coating composition, typically in a concentration of between 2.0 and 20.0% by weight.

These polymers are usually prepared by the (thermal) free radical polymerization of blends of monomers including, styrene, butyl (meth)acrylate, ethyl (meth)acrylate, methyl (meth)acrylate, and/or isobutyl (meth)acrylate. Examples of acrylic polymers include those supplied from Dianal, Elvacite Rohm and Haas and DSM.

The acrylic polymer preferably has an average molecular weight of less than 20,000 g/mole and advantageously less than 10,000 g/mole.

Finally the ink and coating compositions are preferably essentially free of any solvent. However, if required, the ink or coating compositions can be diluted with either organic or aqueous solvents. However, the preferred maximum amount of any solvent is 10.0% by weight.

The inks and coating compositions can be energy-cured by any means. Energy-curing refers to the cure of compositions of the current invention when exposed to various electromagnetic radiation sources producing an actinic effect. Such sources include electron-beam, UV-light, visible-light, IR and microwave.

Where the inks and coating compositions are cured under the preferred action of UV light, then UV sources such as; high-voltage mercury bulb, a medium-voltage mercury bulb, a xenon bulb, a carbon arc lamp, a metal halide bulb, a UV-LED lamp or sunlight, can be used.

Usually Piezo inkjet printheads are preferred for the delivery of inks and coating compositions. However, other printhead technologies, such as continuous inkjet and thermal inkjet may also be used.

The ink and coating compositions can be applied to the non-food contact surface of food packaging including primary and secondary food packaging. The ink and coating compositions and inks of the present invention can be applied to the surface of a plastic film, paper or paperboard substrate.

The plastic film can be, for example, any of the following: polyester, polyethylene, polypropylene, polyamide, poly(lactic acid), a cellulose film and any coated or pretreated film thereof.

The plastic film can be of a flexible or rigid type and can have a thickness of less than or greater than 100µm. A printed plastic film may subsequently be laminated to a further plastic film, to form a printed laminate film suitable for food packaging. A printed paper or paperboard substrate may subsequently be laminated to a further plastic film, to form a printed laminate suitable for food packaging.

The invention is further described by the examples given below.

### EXAMPLES

### Example 1:

The following examples illustrate specific aspects of the present invention and are not intended to limit the scope thereof in any respect and should not be so construed.

### Ink Preparation

The inks were prepared by mixing the pigment dispersion with the ink components using a Silverson type disperser for 20 minutes. The inks were then filtered to remove any oversized particles that might be present in the ink.

### Viscosity Measurements

The viscosities of the inks were measured using a Brookfield DV-II+ Pro Viscometer equipped with Spindle no.18, at 100rpm. In order to be suitable for jetting, it is preferred that the inks of the present invention have a viscosity ≤ 15.0 mPa.s at 50°C, more preferably ≤ 12.0 mPa.s at 50°C.

### Curing the Inks for Extraction/Migration Testing

The inks were applied to 36µm Melinex S (a polyester film) at 12µm and then cured at 200 mJ/cm², using a Fusion UV Systems UV-Rig equipped with a medium pressure H-bulb. The belt speed was adjusted to deliver the required UV-dose of 200 mJ/cm², as measured by a calibrated International Light Technologies ILT 490 Profiling Belt Radiometer (covering the UV-A and UV-B ranges).

### Assessing the Level of Extractable Monomer and Photoinitiator Residues

The level of unbound, unreacted monomer in a print was determined by a 'total extraction' test. This test involved soaking 30cm² of the print in 2ml of methanol, containing 0.025% (w/w) of MEHQ (stabilizer) for 24 hours at room temperature before the methanol solution was analyzed by GC-MS. The GC-MS was calibrated with known solutions of the monomers and photoinitiator products and the results are reported as ppb, the equivalent amount of monomer that would be present in 1Kg of food according to the EU packaging model (where it is assumed that 600cm² of substrate is required to package 1Kg of food) if all the unbound monomer in the print were to migrate into and contaminate the food.

### Assessing the Level of Migratable Species

The level of contamination from a print surface was determined by a 'set-off' migration test. This test involved blocking 90cm² of the printed surface to a 30 micron sheet of LDPE (low density poly(ethene)), at 10 tonnes for a period of 72 hours at room temperature and then for a further period of 10 days at 40°C under a load of 5Kg. The poly(ethene) film was then extracted into 2ml of methanol, containing 0.025% (w/w) of MEHQ (stabilizer) for 24 hours before the methanol solution was analyzed by GC-MS. Similarly, the results are reported as ppb, the amount of migratable material that would be present in 1Kg of food according to the EU packaging model, where it is assumed that 600cm² of substrate is required to package 1Kg of food.

### Assessing the Cure Response of the Inks

The cure response of the inks was determined by applying 12µm films to Leneta opacity charts (Form 2A) using calibrated K-Bars (ex. RK Print). The coated charts were then passed through a Fusion UV Systems UV-Rig equipped with a medium pressure H-bulb. The belt speed was adjusted so that the UV-dose, as measured by a calibrated International Light Technologies ILT 490 Profiling Belt Radiometer (covering the UV-A and UV-B ranges), was about 50 mJ/cm². The number of passes through the rig to achieve both surface and through cure were then recorded allowing the UV-dose level to achieve cure to be determined. The surface cure was assessed by gently drawing a cotton wool bud across the surface of the print, full cure being determined as being the point at which no surface defects were observed. Through cure was assessed by dragging a 1.5 mm wide wooden dowel across the surface of the ink with an approximate downward load of about 5 Kg. Through cure was determined as being the point at which the dowel was not able to penetrate through to the underlying surface of the Leneta Chart.

### Assessing the Odor of Cured Inks

Ink prints prepared according to the method described for extraction and migration testing were assessed for their odor. For this, 5 prints of each ink were prepared and then the coated PET films were stacked on top of each other. After 24 hours, prints from the middle of the stack were taken and assessed for odor. A score of 1 to 6 was assigned to each print, where 1 denotes strong, unpleasant odor and 6 denotes insignificant odor emanating from the print.

### Assessing the Adhesion of Inks to Plastic Surfaces

The inks were applied to a polyethylene laminate (ex. Albea) which had previously been treated via corona discharge to the extent that the surface energy was measured at 40-42 Dynes/cm, with Dyne pens (ex. Dyne Technology). The inks were applied at 12 microns and cured at 200 mJ/cm², as previously described.

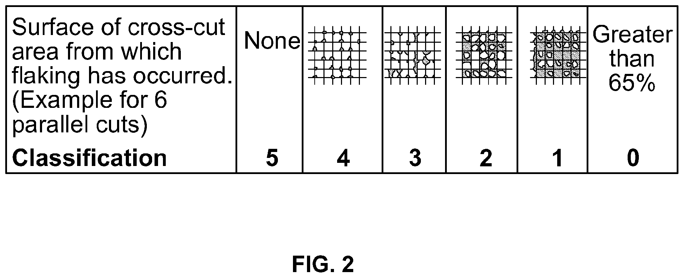

Firstly, the inks were assessed for adhesion according to the Cross Hatch Test according to ASTM D3359-09. A score of 0 (total adhesion loss) to 5 (no adhesion loss) was assigned as a measure of the degree of adhesion (see figure 2).

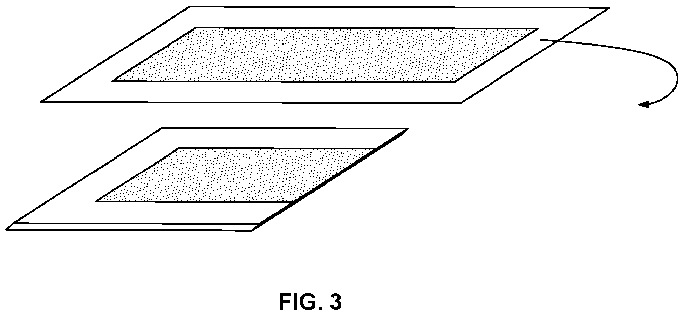

A second adhesion test involved taking the print and folding it (see figure 3), at 0.5cm intervals, for a total of ten folds as depicted below and then performing a tape test across the creases in the print. The adhesion result was reported as the amount of ink remaining on the print.

### The Effect of Using an Aminoacrylate Based on the Michael Addition Reaction Product of Polyacrylate and Ethanolamine

The following ink examples provide comparative examples prepared according to the prior art (EP2671722 (Comparative Example 1 (C1)) and WO2014126720 (Comparative Example 2 (C2))), a further comparative example comprising an aminoacrylate based on the reaction products of alkylamines (C3) and inventive examples (I1 to I6) showing the benefits of the use of an aminoacrylate based on the reaction product of ethanolamine. Furthermore, the impact of introducing a highly alkoxylated monomer according to US Application No. 61971562 is also shown (13 to I6). Note that throughout the text, "C" refers to comparative examples; "I" refers to inventive examples.

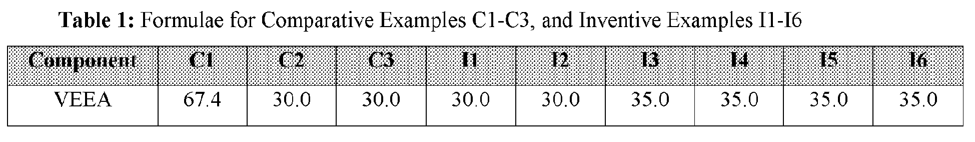

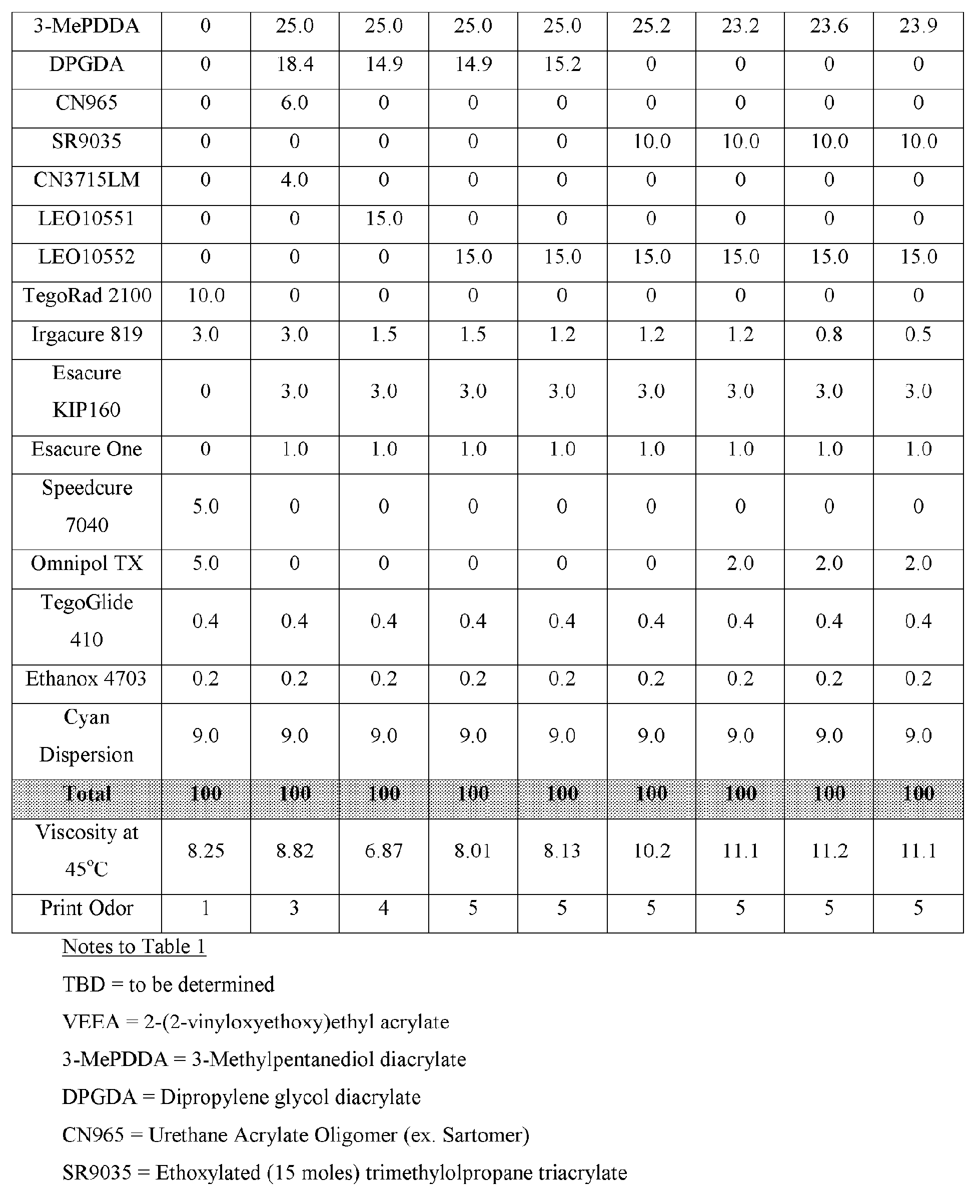

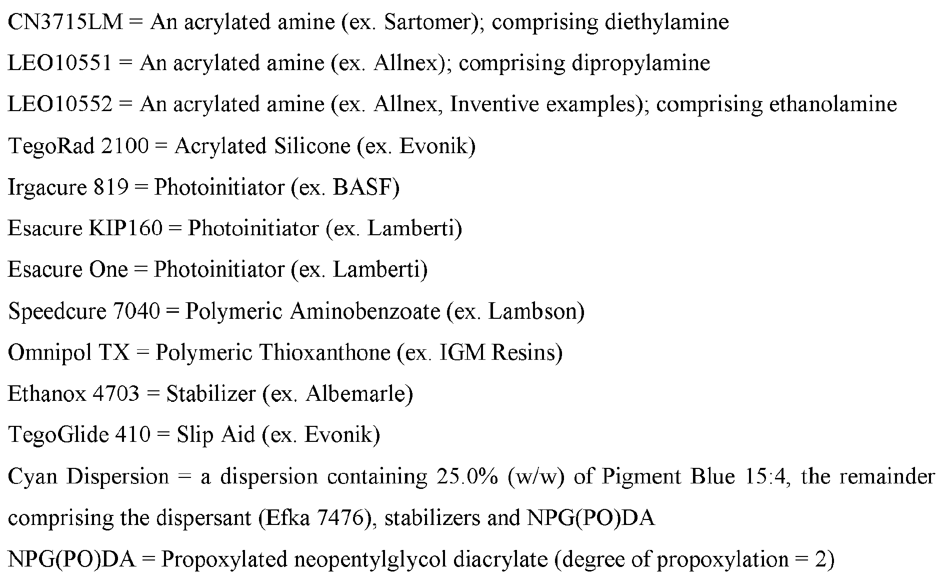

The inks of Table 1 were then assessed for their extractable components according to the procedure outlined above. The results for the extraction test are provided in Table 2.

**Table 2: Extractable Components from Cured Prints of Comparative Examples C1-C3 and Inventive Examples I1-I6**

| **Ink Example** | **Extractable NPG(PO)DA (ppb)** | **Extractable DPGDA (ppb)** | **Extractable 3-MePDDA (ppb)** | **Extractable VEEA (ppb)** | **Extractable Mesitaldehyde (ppb)** |
|---|---|---|---|---|---|
| Example C1 | 297 | - | - | 1,900 | 293 |
| Example C2 | 27 | 22 | 11 | 24 | 490 |
| Example C3 | 46 | 47 | 38 | 86 | 177 |
| Example I1 | 15 | 9 | 7 | 10 | 145 |
| Example I2 | 10 | 7 | 5 | 14 | 108 |
| Example I3 | ND | ND | 2 | 9 | 122 |
| Example I4 | ND | ND | ND | 8 | 170 |
| Example I5 | ND | ND | ND | 7 | 132 |
| Example I6 | ND | ND | ND | 6 | 82 |

| | | | | | |
|---|---|---|---|---|---|
| (ND = Not detected) | | | | | |

**Table 3: Formulae for Ink Examples Comprising 7.5% (w/w) of Aminoacrylate**

| **Component** | **C4** | **C5** | **C6** | **I7** | **I8** |
|---|---|---|---|---|---|
| VEEA | 30.0 | 30.0 | 30.0 | 30.0 | 30.0 |
| 3-MePDDA | 25.0 | 25.0 | 25.0 | 25.0 | 25.0 |
| DPGDA | 21.8 | 21.8 | 21.8 | 21.8 | 21.8 |
| CN3715LM | 7.5 | 0 | 0 | 0 | 0 |
| LEO10551 | 0 | 7.5 | 0 | 0 | 0 |
| LEO10553 | 0 | 0 | 7.5 | 0 | 0 |
| LEO10552 | 0 | 0 | 0 | 7.5 | 0 |
| CN3755 | 0 | 0 | 0 | 0 | 7.5 |
| Irgacure 819 | 0.6 | 0.6 | 0.6 | 0.6 | 0.6 |
| Esacure KIP 160 | 2.0 | 2.0 | 2.0 | 2.0 | 2.0 |
| Esacure One | 2.0 | 2.0 | 2.0 | 2.0 | 2.0 |
| Omnipol TX | 1.5 | 1.5 | 1.5 | 1.5 | 1.5 |
| TegoGlide 410 | 0.4 | 0.4 | 0.4 | 0.4 | 0.4 |
| Ethanox 4703 | 0.2 | 0.2 | 0.2 | 0.2 | 0.2 |
| Cyan Dispersion | 9.0 | 9.0 | 9.0 | 9.0 | 9.0 |
| **Total** | **100** | **100** | **100** | **100** | **100** |
| Theoretical Ink Amine Value (mgKOH/g) | 15.0 | 5.4 | 5.0 | 4.0 | 10.5 |
| Viscosity at 45°C | 6.39 | 6.15 | 6.54 | 6.93 | 7.98 |

**Table 4: Formulae for Ink Examples having an amine value of 6.0mgKOH/g**

| **Component** | **C7** | **C8** | **I9** | **I10** |
|---|---|---|---|---|
| VEEA | 25.0 | 25.0 | 25.0 | 25.0 |
| 3-MePDDA | 30.0 | 30.0 | 30.0 | 30.0 |
| DPGDA | 26.4 | 21.4 | 18.1 | 25.1 |
| CN3715LM | 3.0 | 0 | 0 | 0 |
| LEO10551 | 0 | 8.0 | 0 | 0 |
| LEO10552 | 0 | 0 | 11.3 | 0 |
| CN3755 | 0 | 0 | 0 | 4.3 |
| Irgacure 819 | 1.0 | 1.0 | 1.0 | 1.0 |
| Esacure KIP 160 | 2.0 | 2.0 | 2.0 | 2.0 |
| Esacure One | 2.0 | 2.0 | 2.0 | 2.0 |
| Omnipol TX | 1.0 | 1.0 | 1.0 | 1.0 |
| TegoGlide 410 | 0.4 | 0.4 | 0.4 | 0.4 |
| Ethanox 4703 | 0.2 | 0.2 | 0.2 | 0.2 |
| Cyan Dispersion | 9.0 | 9.0 | 9.0 | 9.0 |
| **Total** | **100** | **100** | **100** | **100** |
| Viscosity at 45°C | 5.58 | 6.21 | 7.68 | 6.81 |

Table 5 provides the test results, other than extractables, for the examples of Tables 3 and 4.

**Table 5: Test Results for the Inks of Tables 3 and 4**

| **Test** | **C4** | **C5** | **C6** | **C7** | **C8** | **I7** | **I8** | **I9** | **I10** |
|---|---|---|---|---|---|---|---|---|---|
| Print Odour | 3 | 4 | 4 | 3 | 4 | 5 | 5-6 | 5 | 5 |
| Through Cure (No. of passes at 50 mJ/cm²) | 3 | 2 | 2 | 2-3 | 2 | 1-2 | 1-2 | 1 | 1-2 |
| Surface Cure (No. of passes at 50 mJ/cm²) | 3-4 | 3 | 3 | 3 | 3 | 2-3 | 3 | 2-3 | 2 |
| Cross-Hatch Adhesion | 2 | 3 | 3 | 3 | 3 | 4 | 4 | 4 | 4 |
| Crease Adhesion Test (% Ink Remaining) | 10 | 25 | 40 | 40 | 40 | 60 | 80 | 80 | 80 |

| | | | | | | | | | |
|---|---|---|---|---|---|---|---|---|---|
| TBD = to be determined | | | | | | | | | |

The results provided in Table 5 show that inks comprising aminoacrylates of the current invention have faster cure responses, produce prints of lower odor and achieve superior adhesion compared with the comparative ink examples comprising aminoacrylates produced by the Michael addition reaction of polyacrylates with alkylamines.

Table 6 provides the results of the extraction test carried out on the examples of tables 3 and 4.

**Table 6: Extraction Results for the Ink Compositions of Tables 3 and 4**

| **Ink Example** | **Extractable NPG(PO)DA (ppb)** | **Extractable DPGDA (ppb)** | **Extractable 3-MePDDA (ppb)** | **Extractable VEEA (ppb)** | **Extractable Mesitaldehyde (ppb)** |
|---|---|---|---|---|---|
| Example C4 | 260 | 159 | 122 | 267 | 39 |
| Example C5 | 342 | 152 | 138 | 73 | 25 |
| Example C6 | 203 | 76 | 67 | 32 | 21 |
| Example I7 | 123 | 50 | 44 | 22 | 23 |
| Example I8 | 28 | 9 | 7 | 9 | 29 |
| Example C7 | 275 | 160 | 149 | 69 | 49 |
| Example C8 | 115 | 37 | 37 | 13 | 45 |
| Example I9 | 15 | 4 | 4 | 2 | 38 |
| Example I10 | 25 | 10 | 6 | 3 | 44 |

The results of table 6 show that ink compositions prepared according to the invention are faster curing, produce prints of lower odour and result in prints having lower levels of extractable monomer. Furthermore, Inventive examples 17 and I10 provide superior adhesion to the polyethylene laminate than do the comparative examples C4, C5 C6, C7 and C8.

Inventive examples 13 to I6 showed the benefit of the inclusion of the highly alkoxylated monomer, ethoxylated (15 moles) trimethylolpropane triacrylate in reducing the amount of extractable monomer. Table 7 provides the compositions of further inventive examples incorporating aminoacrylates of the present invention with the either of the highly alkoxylated monomers ethoxylated (15 moles) trimethylolpropane triacrylate (SR9035, ex. Sartomer) or polyethyleneglycol (400) diacrylate (SR344, ex. Sartomer). Table 7 also provides the viscosity for these inks along with the results of the odour and adhesion tests.

**Table 7: Formulae for Inventive Examples Comprising Aminoacrylates of the Invention, Highly alkoxylated Monomers and having Acyl Phosphine Oxide Photoinitiator Concentrations less than 1.0% (w/w).**

| **Component** | **I11** | **I12** | **I13** | **I14** | **I15** | **I16** | **I17** | **I18** |
|---|---|---|---|---|---|---|---|---|
| VEEA | 35.0 | 40.0 | 50.0 | 40.0 | 40.0 | 40.0 | 40.0 | 40.0 |
| 3-MePDDA | 26.4 | 18.4 | 12.4 | 30.4 | 29.8 | 27.1 | 24.4 | 23.8 |
| DPGDA | 0 | 0 | 0 | 0 | 0 | 0 | 0 | 0 |
| SR344 | 0 | 20.0 | 10.0 | 0 | 0 | 0 | 0 | 0 |
| SR9035 | 8.0 | 0 | 0 | 8.0 | 8.0 | 8.0 | 8.0 | 8.0 |
| CN3755 | 0 | 6.0 | 12.0 | 6.0 | 6.0 | 9.0 | 12.0 | 12.0 |
| LEO10552 | 15.0 | 0 | 0 | 0 | 0 | 0 | 0 | 0 |
| Irgacure 819 | 0.5 | 0.5 | 0.5 | 0.5 | 0.5 | 0.5 | 0.5 | 0.5 |
| Esacure KIP160 | 2.0 | 2.0 | 2.0 | 2.0 | 2.0 | 2.0 | 2.0 | 2.0 |
| Esacure One | 2.0 | 2.0 | 2.0 | 2.0 | 2.0 | 2.0 | 2.0 | 2.0 |
| Omnipol TX | 1.5 | 1.5 | 1.5 | 1.5 | 1.5 | 1.5 | 1.5 | 1.5 |
| TegoGlide 410 | 0.4 | 0.4 | 0.4 | 0.4 | 1.0 | 0.7 | 0.4 | 1.0 |
| Ethanox 4703 | 0.2 | 0.2 | 0.2 | 0.2 | 0.2 | 0.2 | 0.2 | 0.2 |
| Cyan Dispersion | 9.0 | 9.0 | 9.0 | 9.0 | 9.0 | 9.0 | 9.0 | 9.0 |
| **Total** | **100** | **100** | **100** | **100** | **100** | **100** | **100** | **100** |
| Viscosity at 45°C (mPa.s) | 10.6 | 9.6 | 9.75 | 8.01 | 8.19 | 9.09 | 10.5 | 11.1 |
| Print Odour | 5 | 5 | 5 | 5 | 5 | 5 | 5 | 5 |
| Cross-Hatch Adhesion | 5 | 5 | 5 | 5 | 5 | 5 | 5 | 5 |
| Crease Adhesion Test (% Ink Remaining) | 90% | 95% | 100% | 95% | 100% | 100% | 95% | 100% |

The results in Table 7 demonstrate that ink compositions prepared according to the invention and further comprising highly alkoxylated monomers deliver very low odour from cured prints and achieve excellent adhesion. Inventive example I11 was also applied to Fasson PE85 (white), a polyolefinic self-adhesive label, 12 micron Melinex 813, and a corona discharge treated 50 micron Melinex S film. In all 3 cases the ink passed the cross-hatch adhesion test, with a rating of 5.

Table 8 provides the results for the extraction testing on Inventive example I11 to I18

**Table 8: Extraction Results for the Inventive Examples I11 to I18**

| **Ink Example** | **Extractable NPG(PO)DA (ppb)** | **Extractable 3-MePDDA (ppb)** | **Extractable VEEA (ppb)** | **Extractable Mesitaldehyde (ppb)** |
|---|---|---|---|---|
| Example I11 | ND | ND | 3 | 20 |
| Example I12 | ND | ND | 3 | 22 |
| Example I13 | ND | ND | 8 | 28 |
| Example I14 | 3 | 2 | 4 | 20 |
| Example I15 | 3 | 2 | 4 | 18 |
| Example I16 | 2 | 1 | 4 | 22 |
| Example I17 | ND | ND | 4 | 24 |
| Example I18 | ND | 1 | 5 | 21 |

The results in Table 8 show that very low levels of extractable monomer can be achieved with a total photoinitiator concentration of only 6.0% (w/w) of the total ink composition. Furthermore, for Inventive examples I11 to 118 only 0.5% of the acyl phosphine oxide photoinitiator, Irgacure 819, was used. This ensured that low levels of the photodecomposition product mesitaldehyde were achieved in the cured print samples.

Comparative Example C2 and Inventive Example I11 were tested for their set-off migratables according to the procedure outlined above and the results are given in Table 9.

**Table 9: Set-Off Migration Results for Comparative Example C2 and Inventive Example I11**

| **Ink Example** | **Extractable NPG(PO)DA (ppb)** | **Extractable DPGDA (ppb)** | **Extractable 3- MePDDA (ppb)** | **Extractable VEEA (ppb)** | **Extractable Mesitaldehyde (ppb)** |
|---|---|---|---|---|---|
| Example C2 | 2 | 4 | 4 | 6 | 65 |
| Example I11 | ND | ND | ND | ND | 4 |

The results in table 9 demonstrate that mesitaldehyde, the photodecomposition byproduct from acylphosphine oxide type photoinitiators, is highly prone to migrating from the cured ink into plastic films that come into contact with the cured ink. It confirms the benefits achievable with compositions prepared according to the invention in limiting the concentration of such photoinitiators in UV-curable inks intended for the printing of food packaging, pharmaceutical packaging, cosmetics and personal care packaging and other sensitive applications which require the need for prints having not only low odor but low levels of unbound material that could otherwise migrate from the print and cause unwanted contamination.

Note: Comparative Example C2 and Inventive Example I11 were jetted from a Xaar 1002 printhead running at 45°C, with no clogging or mis-directionality jetting problems, even after extended periods of continuous printing.

The present invention has been described in detail, including the preferred embodiments thereof. However, it will be appreciated that those skilled in the art, upon consideration of the present disclosure, may make modifications and/or improvements on this invention that fall within the scope and spirit of the invention.

## Claims

1. An energy curable inkjet ink or coating composition comprising
a) at least one aminoacrylate and
b) and at least one photoinitiator; wherein the photoinitiator comprises a difunctional, multifunctional, oligomeric and/or polymeric photoinitiator,
wherein the total photoinitiator content is less than 12.0% by weight and the total acyl phosphine oxide photoinitiator content is less than 4.0% by weight;
wherein the ink or coating composition comprises less than 20% by weight of monofunctional monomer and
wherein the aminoacrylate is prepared by the Michael addition reaction of a polyacrylate with an alkanolamine having the formula:
HN(R¹)(CₙH₂ₙOH)
wherein R¹ is H or (CₘH₂ₘX);
X is H or OH;
m is 1 to 6 and n is 1 to 6.

2. An ink or coating composition according to claim 1 wherein (i) the alkanolamine is selected from ethanolamine, diethanolamine, propanolamine and/or N -methylethanolamine; and/or (ii) the polyacrylate is selected from hexanediol diacrylate, 3-methylpentanediol diacrylate, dipropylene glycol diacrylate, tripropyleneglycol diacrylate, ethoxylated (3 moles) trimethylolpropane triacrylate, propoxylated (3 moles) glyceryl triacrylate, neopentyl glycol diacrylate, decanediol diacrylate, ethoxylated (2 moles) hexanediol diacrylate, propoxylated (2 moles) neopentyl glycol diacrylate and/or propoxylated (3 moles) trimethylolpropane triacrylate; and/or (iii) the acyl phosphine oxide photoinitiator is phenyl bis(2,4,6-trimethylbenzoyl) phosphine oxide.

3. An ink or coating composition according to any one of the preceding claims having an amine value of greater than 3.0 mgKOH/g, preferably greater than 5.0 mgKOH/g.

4. An ink or coating composition according to any one of the preceding claims wherein the total acyl phosphine oxide photoinitiator content is less than 1.5% by weight, preferably less than 1.0% by weight.

5. An ink or coating composition according to any one of the preceding claims wherein the total photoinitiator content is less than 6.0% by weight, preferably less than 5.0% by weight.

6. An ink or coating composition according to any one of the preceding claims wherein the photoinitiator comprises a difunctional hydroxyketone photoinitiator with the following chemical structures; 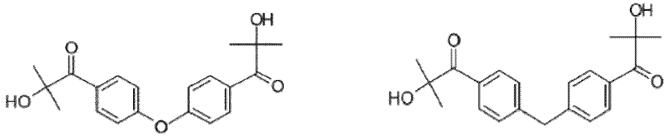 or
wherein the photoinitiator comprises an oligomeric hydroxyketone photoinitiator with the following chemical structure;

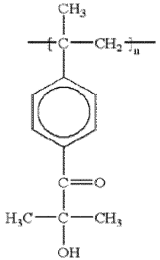

7. An ink or coating composition according to any one of the preceding claims wherein the content of polymeric photoinitiators is less than 3.0% by weight, preferably wherein the polymeric photoinitiator has the following structure;

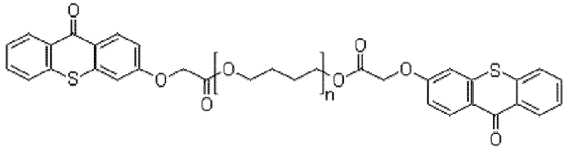

8. An ink or coating composition according to any one of the preceding claims further comprising at least one ethylenically unsaturated monomer or oligomer, preferably wherein the monomers or oligomers comprise at least one acrylate, methacrylate or vinyl ether polymerizable group.

9. An ink or coating composition according to any one of the preceding claims comprising less than 10% by weight of any mono functional monomer, preferably less than 5% by weight of any monofunctional monomer.

10. An ink or coating composition according to claim 8 or claim 9 wherein the polymerizable monomers or oligomers have an alkoxylated chain and at least 2 polymerizable groups per molecule wherein the degree of alkoxylation per polymerizable group is 2 or greater, preferably wherein the polymerizable monomer or oligomer is ethoxylated, propoxylated or butoxylated.

11. An ink or coating composition according to claim 10 wherein the polymerizable monomer or oligomer is an acrylate adduct of the ethoxylated or propoxylated derivative of trimethylol propane, pentaerythritol, dipentaerythritol, glycerol, neopentyl glycol, ethylene glycol, propylene glycol or bisphenol.

12. An ink or coating composition according to claim 10 wherein the monomer is an ethoxylated or propoxylated trimethylolpropane triacrylate, polyethylene glycol diacrylate or polypropylene glycol diacrylate.

13. An ink or coating composition to any one of the preceding claims wherein the ink or coating composition is UV curable.

14. An ink or coating composition according to any one of the preceding claims having a viscosity of less than or equal to 15.0 mPa.s at 50°C, preferably less than or equal to 12.0 mPa.s at 50°C.

15. A coated article comprising a cured ink or coating composition according to any one of the preceding claims on the surface of the article, preferably wherein the article is a food package, a pharmaceutical package, or a cosmetic or personal care package.

16. A process for preparing a coated article comprising
a) applying the ink or coating composition according to any one of claims 1 to 14 to the surface of the article and
b) curing the ink or coating composition; preferably wherein the curing is carried out using UV-light and wherein the total UV dose is less than 400 mJ/cm².

## Patentansprüche

1. Energiehärtbare Tintenstrahltinte oder Beschichtungszusammensetzung, umfassend
a) mindestens ein Aminoacrylat und
b) und mindestens einen Photoinitiator;
wobei der Photoinitiator einen difunktionellen, multifunktionellen, oligomeren und/oder polymeren Photoinitiator umfasst,
wobei der Gesamtphotoinitiatorgehalt weniger als 12,0 Gew.-% beträgt und der Gesamt-Acylphosphinoxid-Photoinitiatorgehalt weniger als 4,0 Gew.-% beträgt; wobei die Tinte oder Beschichtungszusammensetzung zu weniger als 20 Gew.-% monofunktionelles Monomer umfasst und
wobei das Aminoacrylat durch die Michael-Additionsreaktion eines Polyacrylats mit einem Alkanolamin mit der folgenden Formel hergestellt wird:
HN(R¹)(CₙH₂ₙOH)
wobei R¹ für H oder (CₘH₂ₘX) steht;
X für H oder OH steht;
m 1 bis 6 ist und n 1 bis 6 ist.

2. Tinte oder Beschichtungszusammensetzung nach Anspruch 1, wobei (i) das Alkanolamin ausgewählt ist aus Ethanolamin, Diethanolamin, Propanolamin und/oder N-Methylethanolamin; und/oder (ii) das Polyacrylat ausgewählt ist aus Hexandioldiacrylat, 3-Methylpentandioldiacrylat, Dipropylenglycoldiacrylat, Tripropylenglycoldiacrylat, ethoxyliertem (3 Mol) Trimethylolpropantriacrylat, propoxyliertem (3 Mol) Glyceryltriacrylat, Neopentylglycoldiacrylat, Decandioldiacrylat, ethoxyliertem (2 Mol) Hexandioldiacrylat, propoxyliertem (2 Mol) Neopentylglycoldiacrylat und/oder propoxyliertem (3 Mol) Trimethylolpropantriacrylat; und/oder (iii) der Acylphosphinoxid-Photoinitiator Phenylbis(2,4,6-trimethylbenzoyl)phosphinoxid ist.

3. Tinte oder Beschichtungszusammensetzung nach einem der vorstehenden Ansprüche mit einem Aminwert von über 3,0 mgKOH/g, vorzugsweise über 5,0 mgKOH/g.

4. Tinte oder Beschichtungszusammensetzung nach einem der vorstehenden Ansprüche, wobei der Gesamt-Acylphosphinoxid-Photoinitiatorgehalt weniger als 1,5 Gew.-%, vorzugsweise weniger als 1,0 Gew.-% beträgt.

5. Tinte oder Beschichtungszusammensetzung nach einem der vorstehenden Ansprüche, wobei der Gesamtphotoinitiatorgehalt weniger als 6,0 Gew.-%, vorzugsweise weniger als 5,0 Gew.-% beträgt.

6. Tinte oder Beschichtungszusammensetzung nach einem der vorstehenden Ansprüche, wobei der Photoinitiator einen difunktionellen Hydroxyketon-Photoinitiator mit den folgenden chemischen Strukturen umfasst: 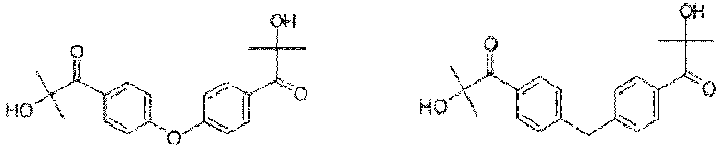 oder wobei der Photoinitiator einen oligomeren Hydroxyketon-Photoinitiator mit der folgenden chemischen Struktur umfasst:

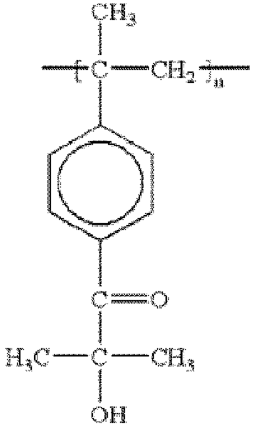

7. Tinte oder Beschichtungszusammensetzung nach einem der vorstehenden Ansprüche, wobei der Gehalt an polymeren Photoinitiatoren weniger als 3,0 Gew.-% beträgt, wobei der polymere Photoinitiator vorzugsweise die folgende Struktur aufweist:

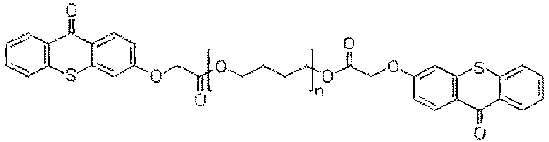

8. Tinte oder Beschichtungszusammensetzung nach einem der vorstehenden Ansprüche, ferner umfassend mindestens ein ethylenisch ungesättigtes Monomer oder Oligomer, wobei die Monomere oder Oligomere vorzugsweise mindestens eine polymerisierbare Acrylat-, Methacrylat- oder Vinylether-Gruppe umfassen.

9. Tinte oder Beschichtungszusammensetzung nach einem der vorstehenden Ansprüche, umfassend zu weniger als 10 Gew.-% ein beliebiges monofunktionelles Monomer, vorzugsweise zu weniger als 5 Gew.-% ein beliebiges monofunktionelles Monomer.

10. Tinte oder Beschichtungszusammensetzung nach Anspruch 8 oder Anspruch 9, wobei die polymerisierbaren Monomere oder Oligomere eine alkoxylierte Kette und mindestens 2 polymerisierbare Gruppen pro Molekül aufweisen, wobei der Alkoxylierungsgrad pro polymerisierbare Gruppe 2 oder mehr beträgt, wobei das polymerisierbare Monomer oder Oligomer vorzugsweise ethoxyliert, propoxyliert oder butoxyliert ist.

11. Tinte oder Beschichtungszusammensetzung nach Anspruch 10, wobei das polymerisierbare Monomer oder Oligomer ein Acrylataddukt des ethoxylierten oder propoxylierten Derivats von Trimethylolpropan, Pentaerythritol, Dipentaerythritol, Glycerin, Neopentylglycol, Ethylenglycol, Propylenglycol oder Bisphenol ist.

12. Tinte oder Beschichtungszusammensetzung nach Anspruch 10, wobei das Monomer ein ethoxyliertes oder propoxyliertes Trimethylolpropantriacrylat, Polyethylenglycoldiacrylat oder Polypropylenglycoldiacrylat ist.

13. Tinte oder Beschichtungszusammensetzung nach einem der vorstehenden Ansprüche, wobei die Tinte oder Beschichtungszusammensetzung UV-härtbar ist.

14. Tinte oder Beschichtungszusammensetzung nach einem der vorstehenden Ansprüche mit einer Viskosität von weniger als oder gleich 15,0 mPa.s bei 50 °C, vorzugsweise weniger als oder gleich 12,0 mPa.s bei 50 °C.

15. Beschichteter Artikel, umfassend eine gehärtete Tinte oder Beschichtungszusammensetzung nach einem der vorstehenden Ansprüche auf der Oberfläche des Artikels, wobei der Artikel vorzugsweise eine Lebensmittelverpackung, eine pharmazeutische Verpackung oder eine Kosmetik- oder Körperpflegeverpackung ist.

16. Verfahren zum Herstellen eines beschichteten Artikels, umfassend
a) Aufbringen der Tinte oder Beschichtungszusammensetzung nach einem der Ansprüche 1 bis 14 auf die Oberfläche des Artikels und
b) Härten der Tinte oder Beschichtungszusammensetzung;
wobei das Härten vorzugsweise unter Verwendung von UV-Licht ausgeführt wird und wobei die Gesamt-UV-Dosis weniger als 400 mJ/cm² beträgt.

## Revendications

1. Composition d'encre pour jet d'encre ou de revêtement, durcissable par énergie comprenant
a) au moins un aminoacrylate et
b) et au moins un photoinitiateur ; le photoinitiateur comprenant un photoinitiateur difonctionnel, multifonctionnel, oligomérique et/ou polymérique,
la teneur totale en photoinitiateur étant inférieure à 12,0 % en poids et la teneur totale en photoinitiateur de type oxyde d'acylphosphine étant inférieure à 4,0 % en poids ;
la composition d'encre ou de revêtement comprenant moins de 20 % en poids de monomère monofonctionnel et
l'aminoacrylate étant préparé par la réaction d'addition de Michael d'un polyacrylate avec une alcanolamine possédant la formule :
**HN(R¹)(CₙH₂ₙOH)**
R¹ étant H (CₘH₂ₘX) ;
X étant H ou OH ;
m étant 1 à 6 et n étant 1 à 6.

2. Composition d'encre ou de revêtement selon la revendication 1, (i) l'alcanolamine étant choisie parmi l'éthanolamine, la diéthanolamine, la propanolamine et/ou la N-méthyléthanolamine ; et/ou (ii) le polyacrylate étant choisi parmi le diacrylate d'hexanediol, le diacrylate de 3-méthylpentanediol, le diacrylate de dipropylèneglycol, le diacrylate de tripropylèneglycol, le triacrylate de triméthylolpropane éthoxylé (3 moles), le triacrylate de glycéryle propoxylé (3 moles), le diacrylate de néopentylglycol, le diacrylate de décanediol, le diacrylate d'hexanediol éthoxylé (2 moles), le diacrylate de néopentylglycol propoxylé (2 moles) et/ou le triacrylate de triméthylolpropane propoxylé (3 moles) ; et/ou (iii) le photoinitiateur de type oxyde d'acylphosphine étant l'oxyde de phényl-bis (2, 4, 6-triméthylbenzoyl)phosphine.

3. Composition d'encre ou de revêtement selon l'une quelconque des revendications précédentes possédant une valeur d'amine supérieure à 3,0 mg de KOH/g, préférablement supérieure à 5,0 mg de KOH/g.

4. Composition d'encre ou de revêtement selon l'une quelconque des revendications précédentes, la teneur totale en photoinitiateur de type oxyde d'acylphosphine étant inférieure à 1,5 % en poids, préférablement inférieure à 1,0 % en poids.

5. Composition d'encre ou de revêtement selon l'une quelconque des revendications précédentes, la teneur totale en photoinitiateur étant inférieure à 6,0 % en poids, préférablement inférieure à 5,0 % en poids.

6. Composition d'encre ou de revêtement selon l'une quelconque des revendications précédentes, le photoinitiateur comprenant un photoinitiateur difonctionnel de type hydroxycétone comportant les structures chimiques suivantes ; 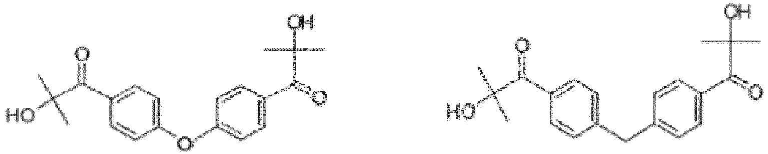 ou le photoinitiateur comprenant un photoinitiateur oligomérique de type hydroxycétone comportant la structure chimique suivante ;

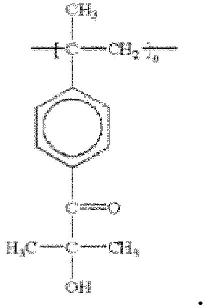

7. Composition d'encre ou de revêtement selon l'une quelconque des revendications précédentes, la teneur en photoinitiateurs polymériques étant inférieure à 3,0 % en poids, préférablement le photoinitiateur polymérique possédant la structure suivante ;

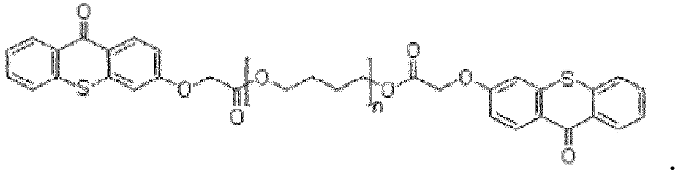

8. Composition d'encre ou de revêtement selon l'une quelconque des revendications précédentes comprenant en outre au moins un monomère ou oligomère éthyléniquement insaturé, préférablement les monomères ou les oligomères comprenant au moins un groupe polymérisable de type acrylate, méthacrylate ou éther de vinyle.

9. Composition d'encre ou de revêtement selon l'une quelconque des revendications précédentes comprenant moins de 10 % en poids d'un quelconque monomère monofonctionnel, préférablement moins de 5 % en poids d'un quelconque monomère monofonctionnel.

10. Composition d'encre ou de revêtement selon la revendication 8 ou la revendication 9, les monomères ou les oligomères polymérisables possédant une chaîne alcoxylée et au moins 2 groupes polymérisables par molécule, le degré d'alcoxylation par groupe polymérisable étant de 2 ou plus, préférablement le monomère ou l'oligomère polymérisable étant éthoxylé, propoxylé ou butoxylé.

11. Composition d'encre ou de revêtement selon la revendication 10, le monomère ou 1'oligomère polymérisable étant un adduit d'acrylate du dérivé éthoxylé ou propoxylé du triméthylolpropane, du pentaérythritol, du dipentaérythritol, du glycérol, du néopentylglycol, de l'éthylèneglycol, du propylèneglycol ou du bisphénol.

12. Composition d'encre ou de revêtement selon la revendication 10, le monomère étant un triacrylate de triméthylolpropane, un diacrylate de polyéthylèneglycol ou un diacrylate de polypropylèneglycol, éthoxylé ou propoxylé.

13. Composition d'encre ou de revêtement selon l'une quelconque des revendications précédentes, la composition d'encre ou de revêtement étant durcissable par les UV.

14. Composition d'encre ou de revêtement selon l'une quelconque des revendications précédentes possédant une viscosité inférieure ou égale à 15,0 mPa.s à 50 °C, préférablement inférieure ou égale à 12,0 mPa.s à 50 °C.

15. Article revêtu comprenant une composition d'encre ou de revêtement durcie selon l'une quelconque des revendications précédentes sur la surface de l'article, préférablement l'article étant un emballage alimentaire, un emballage pharmaceutique, ou un emballage cosmétique ou de soin personnel.

16. Procédé pour la préparation d'un article revêtu comprenant
a) l'application de la composition d'encre ou de revêtement selon l'une quelconque des revendications 1 à 14 à la surface de l'article et
b) le durcissement de la composition d'encre ou de revêtement ; préférablement le durcissement étant mis en œuvre à l'aide de lumière UV et la dose totale d'UV étant inférieure à 400 mJ/cm².
